# EUROPEAN PATENT APPLICATION

(11) **EP 3 321 878 A1**
(43) Date of publication of application: **16.05.2018**
(21) Application number: 17203376.3
(22) Date of filing: 09.06.2012
(51) Int. Cl.: G06Q 50/22, G06F 19/00

(54) **METHOD AND APPARATUS FOR MONITORING MEDICATION ADHERENCE**

(30) Priority: 10.06.2011 US 201161495415 P; 24.07.2011 US 201113189518
(62) Divisional of application: 12796828.7
(71) Applicant: Ai Cure Technologies, Inc., New York, NY 10010 (US)
(72) Inventor: HANINA, Adam, New York, NY New York 10024 (US); KESSLER, Gordon, Mt. Kisco, NY New York 10549 (US); GUAN, Lei, Harrison, NJ New Jersey 07021 (US)
(74) Representative: Fox-Male, Nicholas Vincent Humbert

(57) **Abstract**

A method and apparatus for monitoring medication adherence. The method includes the steps of determining a present adherence state of a patient, receiving video analysis information reporting on a medication administration session, and determining a next adherence state of a patient based upon the present adherence state of the patient and the video analysis information.

## Description

### Cross Reference to Related Applications

This application claims the benefit of US Provisional Patent Application Serial No. 61/495,415 filed June 10, 2011 to Hanina et al., titled Method and Apparatus for Monitoring Medication Adherence, and claims priority to US Patent Application Serial No. 13/189,518 filed July 24, 2011 to Hanina et al., titled Method and Apparatus for Monitoring Medication Adherence, the entire contents of each of these applications being incorporated herein by reference.

### Field of the Invention

This invention relates generally to the monitoring of patient medication adherence to a prescribed regimen, and more particularly to organization and automated monitoring of automatically generated patient medication administration data.

### Background of the Invention

The total healthcare cost of drug-related morbidity, including poor adherence, is estimated at $290 billion per year in the US. "National Council on Patient Information & Education. Thinking Outside the Pillbox A System-wide Approach to Improving Patient Medication Adherence for Chronic Disease. *NEHI Research Brief.* August 2009. www.nehi.net/uploads/full_report/pa_issue_brief final.pdf." Treatment of patients with poor adherence can require twice the resources from the healthcare system than treatment of more compliant individuals. "Sokol M, McGuigan K, Verbrugge R, Epstein R. Impact of medication adherence on hospitalization risk and healthcare cost. Med Care. June, 2005;43(6):521-30." Mortality and morbidity rates are much higher for patients who do not follow their prescribed drug therapy, especially for patients suffering from a chronic illness. "Ho P, Magid D, Masoudi F, McClure D, Rumsfeld J. Adherence to cardioprotective medications and mortality among patients with diabetes and ischemic heart disease. BMC Cardiovasc Disord. December, 2006;15;6:48." Currently, 75% of healthcare spending in the US is directed towards treatment of chronic disease. "CDC. Chronic Disease Prevention and Health Promotion. http://www.cdc.gov/chronicdisease/resources/publications/AAG/chronic.htm." These same chronically ill patients who are also nonadherent to their medication prescriptions are twice as likely to be hospitalized. "Kenreigh C, Wagner L. Medication Adherence: A Literature Review 2005. Medscape. 2005. http://www.medscape.com/viewarticle/514164"; and "Sokol M, McGuigan K, Verbrugge R, Epstein R. Impact of medication adherence on hospitalization risk and healthcare cost. Med Care. June, 2005;43(6):521-30." In psychiatric patients in particular, medication nonadherence is among the most common causes of psychotic relapse and rehospitalization. "Marder, Stepher R., Overview of Partial Compliance, J Clin Psychiatry 2003; 64[suppl 16];3-9*."*

Barriers to medication adherence such as the perceived impact of a medicine, knowledge about illness, forgetfulness, or lack of social support, "Friedman et. al.; Voila et al.; Wu et al. Three studies quoted in W.F., Grenard J, McGlynn E.A. A Review of Barriers to Medication Adherence: A Framework for Driving Policy Options. RAND, 2009", help to explain why 75% of Americans do not take their medicine as prescribed. "National Council on Patient Information and Education. Enhancing Prescription Medicine Adherence: A National Action Plan. Bethesda, Md. August, 2007. www.intelecare.com/downloads/ncpie-adherence-report.pdf." Traditional monitoring methods have problems with reliability and cost and generally fail to allow for immediate intervention by a healthcare professional. Pill counting and patient interviews are unreliable ways of measuring medication adherence. "Osterberg L, Blaschke T. Adherence to medication. N Engl J Med. August 4, 2005;353(5):487-497." Self-reporting by individuals, using ePRO diaries, IVRS or web portal communications have also been shown to be untrustworthy as many patients fail to record accurate data. "Simmons M, Nides M, Rand C, Wise R, Tashkin D. Unpredictability of deception in compliance with physician-prescribed bronchodilator inhaler use in a clinical trial. Chest 2000 118:290-295." Technology such as digital pill container caps and smart packaging report only when patients open the medication container and cannot confirm medication administration. Importantly, these methods do not provide timely information sufficient to support care provider intervention. Smart pills, while accurate, are expensive and require the manufacturing process of the medication to be altered to include an RFID or other identification computer chip therein. Even data tools such as electronic health records fail to capture patient behavior such as medication adherence rates despite a new emphasis on meaningful use, fail to perform any useful analysis on captured data, and fail to learn any behavioral patterns to assist in adherence monitoring. "National Cancer Institute. https://www.gem-beta.org/Public/EHRInitiative.aspx?cat=4."

An extremely effective way to confirm medication adherence is through direct observation, i.e. watching a patient take their medication. The WHO's Directly Observed Treatment, short course (DOTs) program has radically improved compliance rates of TB patients. "Stop TB Partnership. The Global Plan to Stop TB, 2006-2015: Actions for life: towards a world free of tuberculosis. Geneva: WHO; 2006. http://whqlibdoc.who.int/publications/2006/9241593997_eng.pdf." Such direct observation is typically employed in phase 1 clinical trials, where assurance of adherence is critical. Unfortunately, the labor-intensive nature of the program is expensive, time consuming and geographically limited, as well as being inconvenient and burdensome to patients.

Dr Lars Osterberg, M.D. and Dr, Terence Blaschke have reported in the New England Journal of Medicine, Adherence to Medication, (N Engl J Med 2005; 353:487-97) 2005 an alarming lack of adherence to required medication protocol, further noting that while the average rates of adherence in clinical trials is categorized as "high", this number still comprises only rates of 43 to 78 percent. Most importantly, the authors note "The ability of physicians to recognize nonadherence is poor, and interventions to improve adherence have had mixed results." Adherence, p. 487.The authors conclude "Poor adherence to medication regimens is common, contributing to substantial worsening of disease, death and increased healthcare costs." Adherence, p. 494. The Trend Repot Series, 2008 Patient Adherence Update: New Approaches for Success, October 2008, report similar discouraging statistics. This broad range may possibly contribute to the public confidence in the FDA approval process and the importance of continued surveillance of a drug throughout the process. Furthermore, it may help to explain why, according to the Journal of the American Medical Association (JAMA May 1, 2002), one out of every five new drugs that comes to market in the US is found to have serious or life-threatening adverse effects - unknown or undisclosed at the time of approval. It is against this backdrop of poor adherence, and potential danger to patients, that the present invention operates.

It has been widely recognized that methods and systems for insuring proper medication ingestion or administration by individuals are very important in defending against unnecessary sickness, deaths and other problems. Giving instructions and then letting patients fend for themselves has been shown not to work particularly well. This is because it is not only the improper ingestion of medicines that is the primary cause of medical danger. Rather, an overall lack of sufficient patient guidance is also part of the problem. Further, the inability to confirm a proper prescription regimen being provided to a user in the first place may cause a number of other problems with the use of such medication.

Traditionally, participants attend introductions and follow ups for clinical trials in-person. Other patients attempting to adhere to a particular medication protocol similarly are given a prescription and a particular set of instructions from a prescribing medical provider or prescribing doctor, and then compliance is measured, typically by counting remaining pills, at a next visit with that prescribing professional. Thus, data collection is similarly limited to patient visits, rather than on a daily basis. Old methods such as patient questioning and pill counting have been proven to be inadequate measures of adherence and offer no information on dose timing and drug holidays (omission of medication for three or more sequential days, for example).

Compliance technologies can increase the statistical power of clinical trials. Through the use of such technology, clinical events can be precisely linked to medication use history. Captured data can be linked to other sources such as EDC, patient diaries and data collected by the physician. Technologies can create many possibilities for remote visits and data capture. While smart packaging technologies exist such as RFID-enabled computer chip technology, smart blister packs and MEMS caps (microprocessor in a bottle cap), they are: a) invasive and need to be physically attached to the medications; b) are non-conclusive regarding compliance - a patient may activate the technology without ingestion of the medication; c) remain largely unadopted in clinical trials by the pharmaceutical and biotech companies due to their high cost; and d) take a longer time to implement. Further, electronic patient diaries allow for ease of entry of data by a patient. These diaries, however, are still subject to issues related to compliance with medication adherence. Thus, even if a patient is meticulous about entering information into the diary, and thus complying with the requirements for data entry, there is still no guarantee that they are properly taking medication at prescribed times.

Jo Carol et al. stated that "The most reliable method for research purposes, although not practical in a clinical setting, may be a combination approach that includes pill counts, patient self-report, and electronic monitoring." (Carol J. et al, Patterns to Antiretroviral Medication, The Value of Electronic Monitoring, AIDS, 17 (12), pp1, 763-767, Oct 2003. Furthermore, it is well known that it is expensive to check up on people and directly monitor medication administration, but studies have shown that care provider intervention has a significant benefit on medication adherence rates and patient behavior. http://www.ahdbonline.com/feature/engaging-providers-medication-adherence-health-plan-case-study. To date, technologies alone have only been used in an attempt to monitor compliance rather than to encourage it. Furthermore, there has been no comprehensive system provided that allows for the management of multiple patients and multiple patient populations. While current technology may allow poor compliers to be recognized, as will be described below, the proposed apparatus and method of the present invention will help to encourage pharmaceutical compliance and tackle some of the problems that are encountered in the clinical trial process in particular, and the medication protocol monitoring problem in general.

A number of systems exist that provide instructions to a user regarding when to take a medication and records when the user indicates that a medication has been taken. US Patent No. 7,359,214 describes such a system. A device is provided that instructs a patient regarding medications to take. Furthermore, the system may provide a method for determining that the prescription is appropriate given the patient's conditions, and other medications he or she may already be taking. The system may monitor the dispensing of medicine in accordance with a predetermined treatment protocol. While such a system provides many improvements for easing a burden on the patient, this system suffers in many ways and in particular in ways relevant to the administration of clinical trials and other active patient monitoring of medication adherence.

Most importantly, this system provides no mechanism for actually confirming that a patient is in fact ingesting or otherwise properly administering medication as required in a clinical drug trial, or as prescribed by a prescribing physician in the case where adherence to a particular regimen may prove to be critical to efficacy of the prescription regimen. Further, while the system may be sufficient for one who is in full possession of their mental faculties, any individual who may have difficulty following directions, or one who is actively avoiding medication may still not be taking required medication after it is dispensed. Thus, participants may be forgetful, visually impaired, or otherwise do not believe in the benefit of taking such medication, and may thus not properly log medication administration. Additionally, the system requires preloading of various medications into a dispenser, and thus likely requires regular visits by an administering manager to be sure appropriate medications are in fact properly loaded therein. It is surely possible that an inexperienced user may place incorrect medications into the device, or may somehow provide incorrect dosages into the device. Still further, for potentially more complex regimens, there is no method provided for insuring that a user is able to follow such a protocol, and to thereafter confirm that the user has in fact taken all required medications in accordance with any provided instructions or the like, or has taken the medications according to one or more specifications or followed suggested procedures. Additionally, there is no method for determining in near real time whether a patient has taken their medication, and does not allow for intervention on the part of a healthcare provider to immediately address adherence issues. Finally, this system is expensive and requires constant maintenance to confirm that the various mechanical parts are in working order.

US Patent Application Serial No. 11/839,723, filed August 16, 2007, titled Mobile Wireless Medication Management System provides a medication management system employing mobile devices and an imaging technology so that a user is able to show a pill to be taken to the system, and the system can then identify the medication. Patient histories are available to an administrator, including various vital signs as measured by the system. Images may also be taken of the patient, provider, medication container or the like. While the system professes to ensure adherence to a protocol, the system only provides such help if requested by a user. There is in fact no particular manner in which to ensure actual adherence (i.e. taking of the medication by a particular person) or the relationship of adherence to the efficacy of the drug over time. Furthermore, the system relies only on a single still image of the medication, thus not being very versatile if the image is poor. When requiring adherence to a predetermined protocol for a clinical trial, this is particularly relevant.

Additionally, existing systems fail to maintain an audit trail for post administration review by a medical official or other clinical trial administrator, and further cannot therefore confirm confirmation of proper medication administration. They also fail to allow for intervention by a healthcare provider on a near real time basis, and indeed fail to properly allow an administrator to monitor a large group of patients efficiently and accurately.

Therefore, it would be desirable to provide a method and apparatus that overcome the drawbacks of the prior art.

### Summary of the Invention

In US Patent Application Serial Nos. 12/620,686 filed November 18, 2009 titled Method and Apparatus for Verification of Medication Administration Adherence; 12/646,383 filed December 23, 2009 titled Method and Apparatus for Verification of Clinical Trial Adherence; 12/646,603 filed December 23, 2009 titled Method and Apparatus for Management of Clinical Trials; and 12/728,721 filed March 22, 2010 titled Apparatus and Method for Collection of Protocol Adherence Data, the entire contents of each of these applications being incorporated herein by reference, as well as in other co-owned applications, the inventors of the present invention have proposed a system and method that allow for complete control and verification of adherence to a prescribed medication protocol or machine or apparatus use in a clinical trial or other setting, whether in a health care provider's care, or when self administered in a homecare situation by a patient. As part of these applications, determination of when a user has taken a pill is an important step in the monitoring process. Further determination of user administration of inhalable, injectable and other medication administration processes may also be provided. These applications also describe a dashboard presented to one or more healthcare providers in order to properly aggregate and review various monitored medication administration sequences for any number of patients.

These applications present the only medication management system that may determine whether a user is actually following a protocol (preferably employing audio/video analysis of captured audio/video information), provide additional assistance to a user, starting with instructions, video instructions, and the like, and moving up to contact from a medication administrator if it is determined that the user would need such assistance in any medical adherence situation, including clinical trial settings, home care settings, healthcare administration locations, such as nursing homes, clinics, hospitals and the like, and in clinical trial settings.

In accordance with various aspects of the present invention, a web-based (or otherwise housed) dashboard may be provided to manage information captured from a computer vision software module that uses webcams to automate the direct observation of medication administration without the need for one on one human supervision. The dashboard will allow healthcare providers to monitor medication adherence rates and interact with patients through a patient issued (or patient owned) webcam enabled laptop, smartphone or other device in the hospital room, at home, or in any other convenient location, and store, monitor and review recorded video data associated with one or more patients using the system. This interactive and functional application is unlike traditional electronic medical records which provide a full medical history but often failing to capture data reflecting crucial health behaviors. The inventive solution offers a clear snapshot of medication adherence behavior both past and present. Healthcare providers may be notified of behavioral trends in medication adherence, receive alerts, and review correlations to medication efficacy and contraindications. Further, healthcare providers may view such information at the patient, small group, or population level, thus allowing for trends to be noticed, yet individual attention to be provided.

Importantly, a user may be able to quickly switch from viewing one patient's profile in the dashboard to viewing entire patient populations in one screen. Summary statistics and demographic information may also be accessible. The system may highlight predictive patterns of behavior and alert care providers to possible "danger zones". Other population based metrics may also be employed. The more data collected, the more efficient the system will be at predicting patterns and risk. The integrated communication platform may allow for patient communication and intervention when appropriate. Interventions may include automated messages (text, audio, visual) on the patient's device triggered by specific events or trends, live phone calls, video conferencing requesting in-person appointments, status updates or other appropriate notifications and contacts. This may encourage adherence to prescribed protocol and reduce expensive hospital readmissions.

Embodiments of the present invention may allow automated direct observation of medication adherence to be used as a population health tool, especially where the risk and cost of patients not taking medication is high and stakeholders have a vested interest in monitoring behavior. The system may help to virtualize the patient, avoid the reliance on self-reporting or direct human supervision, and still allow for intervention when necessary. The system may also work to improve medication adherence. Patient safety and treatment will confidently be assured and fewer supervisory personnel will be needed to supervise much larger patient populations thus reducing overall costs of patient supervision. Summary data of medication adherence rates may provide the basis for intervention and reaction to the medication could lead to a change prescribing practice. This is especially useful for chronic conditions, complex drug regimens, and patients transitioning from an inpatient to an outpatient environment. Faster follow-up in certain at risk populations may reduce rehospitalizations. The system may also have applications in clinical trials, lowering costs and increasing safety and efficacy because of more reliable adherence data. Better regulation can be enforced and action swiftly taken before drugs come to market.

Various embodiments of the present invention will obtain and manage video information of patient medication administration. As described in the above-referenced applications, such video data is captured and analyzed to determine medication adherence. The patient may be provided with immediate feedback related to such administration, and thus a focused application will allow for immediate feedback to the user. Determination of adherence is used to categorize patients into various patient states, and thus allow for reaction of the system to patients in particular patient states. In addition, this video information may be stored for further analysis offline, in a common or remote location, and may employ substantially greater computing power than available on an individual mobile or other device, such as analyzing trends in adherence and other factors over time, as will be described in detail below. These trends may be used to further define patient states. Further information, including other medication administration information such as visual cues, audio cues, side effect information, contraindication information, positive medication effects and the like.

Therefore, various embodiments of the present invention will provide a state machine of the type described below that utilizes audio/video information to offer a population health tool to manage any number of patients, understand their behavior, and communicate and intervene when necessary or desirable. The system further employs machine learning to identify one or more trends and make automated judgments about patient states, as well as an ability to learn and highlight outliers or at risk populations. Thus, based upon captured information, patients may be placed into states that may aid in predicting those patients at risk for future hospitalizations, for example, or other types of situations where a varied intervention strategy may be beneficial. When considering large patient populations, such automated monitoring and categorization allows for monitoring of such patients, allowing managers to direct their attention to patients who might best benefit from such attention, and allowing the system to provide automated intervention when determined to be appropriate. This level of automated, intelligent intervention allows for effective management of patient behavior and medication adherence. Existing systems fail to capture patient behavior. Various embodiments of the inventive solution, including the described state machine, provide data relevant to medication adherence and other medical treatments as opposed to entire patient history, such as in an existing electronic medical record. Furthermore, the system acts as a video repository, recording administration by patients, and thus allowing for future review of such administration sequences by a manager or other healthcare professional if appropriate. Thus, upon determination by the system in a manner noted above, patients in one or more predetermined states may be indicated for such manual review by the manager or other healthcare provider. Finally, the inventive system may be applicable not only to adherence information, but to any patient action or healthcare related treatment to which monitoring may be applicable.

Still other objects and advantages of the invention will in part be obvious and will in part be apparent from the specification and drawings.

The invention accordingly comprises the several steps and the relation of one or more of such steps with respect to each of the others, and the apparatus embodying features of construction, combinations of elements and arrangement of parts that are adapted to affect such steps, all as exemplified in the following detailed disclosure, and the scope of the invention will be indicated in the claims.

### Brief Description of the Drawings

For a more complete understanding of the invention, reference is made to the following description and accompanying drawings, in which:
Figure 1 is a flowchart diagram depicting an embodiment of the invention;
Figure 2 is a flowchart diagram depicting more detailed steps associated with step 110 of Figure 1;
Figure 3 is a flowchart diagram depicting progression of a patient through a plurality of medication administration states in accordance with an embodiment of the invention; and
Figure 4 is an exemplary medication adherence dashboard constructed in accordance with an embodiment of the invention.

### Detailed Description of the Preferred Embodiments

The invention will now be described making reference to the following drawings in which like reference numbers denote like structure or steps.

In accordance with one or more embodiments of the present invention, healthcare providers are provided with access to real-time medication adherence information through a dashboard, allowing for active participation rather than passive observation in medication administration and tracking. The inventive solution combines population health, computer vision, predictive tools based on behavioral markers, and a built-in communication system to monitor and manage patients' medication adherence. The inventive system may provide analysis of medication efficacy and effectiveness in one or more different patient populations, such as by medication types, demographic groups, care provider performance and the like. The system will encourage better compliance and radically improve patient-provider care.

Embodiments of the invention present a patient management solution specifically geared towards medication adherence and other patient activities. Unlike electronic medical records and population health solutions that capture general medical history data and perform no analysis, embodiments of the present invention capture near real-time patient behavior data and may perform analysis, in near real time, and in a more advanced manner in an offline format. One or more embodiments of the present invention comprise computer vision technology to ensure that a patient is accurately recorded taking their medicine by tracking and confirming the patient's actions on the screen, identifying a pill to be taken and confirming the pill is swallowed. Other medication administration sequences may also be observed, such as inhaler apparatuses, injectable apparatuses, and the like. These data points may be saved and analyzed, providing real-time behavioral markers to healthcare providers giving them a much more accurate reflection of patient behavior. Healthcare providers may be notified through the dashboard of potentially nonadherent patients. Further information, such as doctor's prescribing trends and the effect on adherence, effectiveness and adherence in specific patient populations, as well as one or more trends associated with individual or population adherence may be provided.

Intervention techniques including automated or direct individual contact with the patient may be initiated from the dashboard in an automated manner, or at the request of a healthcare provider. Such intervention techniques may range from automated mass messages, to individual, personal text or email messages, to video conference, where appropriate. All messages and other interventions will be stored along with a patient's information so that further follow up task lists can be more easily managed, and so that healthcare provider has complete access to all patient data. Thresholds for use of such intervention strategies may be determined by the healthcare provider or system administrator. This will allow for more immediate intervention by healthcare providers to monitor and aid potentially limitless patient populations and their adherence, intervening only where necessary and likely to be effective. Indirect methods of determining patient adherence typically rely on patient questionnaires and pill counting, or employ more direct monitoring of a patient's actions, including monitoring the time of opening of bottles, dispensing drops, or activating a canister. Each of these methods is passive and only confirms whether a patient has opened a pill bottle or interacted with a device. Real-time patient behavior is not available. Therefore providers cannot react quickly or easily spot behavioral trends and changes in symptoms or side effects.

Embodiments of the present invention link adherence data to medication efficacy and patient safety, allowing for immediate interaction by a healthcare provider to improve medication adherence for a fraction of the cost of true direct observation. One or more embodiments of the present invention contemplate capture, storage and analysis of direct visual information of a patient, including but not limited to medication administration actions, patient appearance and other actions and any other patient information that may be acquired through the visual and other acquisition systems. Automated processing and analysis of acquired patient data allows for direct observation of patients, while greatly reducing the cost by reducing the need for human review of acquired information. Preferably, only when the automated system indicates a need will human review be implemented. However, the full audit trail of time and date stamped audio video captured information may be viewed as desired by a healthcare provider, clinical trial manager or the like at any time via the dashboard.

Understanding and tracking adherence statistics may allow healthcare providers to meet quality metrics, adopt improved care processes, assume risk, and provide incentives for population health and wellness. The inventive population dashboard progresses knowledge of an unsolved area and responds to the need to place a high priority on innovations offering fundamental and applied research for the assistance of chronically ill patients. In addition, the inventive population dashboard will offer significant impact to the area of clinical trials and postmarketing surveillance of medications. Despite the FDA's rigorous preapproval processes, even well executed randomized controlled clinical trials cannot uncover all safety problems or rare serious adverse events either in the general population or sub-populations. Trials are simply not large enough, varied enough, or long enough in duration. It is only through rigorous monitoring of huge numbers of patients that any such rare events may be uncovered. Linking adherence data to such rare events is critical in warning of any such dangers in particular demographic patient populations. The system may be applicable in inpatient, outpatient, disease management, clinical trials, and other patient monitoring scenarios.

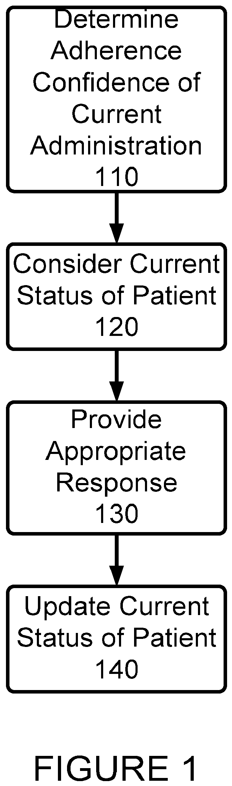

Referring first to Figure 1, a flowchart diagram for depicting an overall system functionality in accordance with an embodiment of the present invention is provided. As is set forth in Figure 1, at step 110, a patient is monitored administering medication (or other individual is monitored administering medication to the patient), and a determination is made as to whether the patient has properly administered such medication in accordance with one or more of the procedures noted in the above-referenced co-assigned patent applications. Other features, such as patient identification, medication identification, and the like may also be implemented in step 110. After such a determination is made, at step 120, a current status of a particular patient is determined. This status preferably includes recent track records for taking medication adherence, number of consecutive missed administrations, if applicable, whether the patient is in a high risk group that is more likely to have adherence problems, whether the particular drug to be administered has a time and/or date critical administration prescription, and the like. Then at step 130, in consideration of the current state of the patient and the results of the monitoring of the current medication administration determination at step 110, an appropriate response is enacted, preferably including an automated reminder to the patient, an automated reminder to a medical professional, a personal contact to the patient from a medical professional or other administrator, encouraging messages congratulating on a correct administration, or no response. Then finally at step 140, the current status of the patient is updated to account for the adherence determination of step 110 and any provided response at step 130.

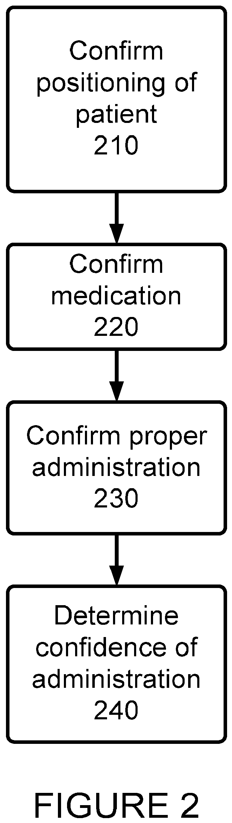

Referring next to Figure 2, a more in depth method for determining the adherence confidence of the current medication administration at step 110 will be provided. As is shown in Figure 2, first at step 210, the position of a patient is confirmed electronically. Then at step 220, the medication to be administered may be requested to be positioned in a particular location in the field of view, and may be confirmed through any number of color, bar coding, marking, shape or other identifiable characteristics. At step 230, proper administration of the medication is monitored, and at step 240, the confidence of administration of the medication is determined. This confidence level may be determined based upon various information, such as time on task, movements by the patient, shadows, poor lighting, or any other environmental or other factor that may decrease the confidence with which an automated machine vision system may confirm medication administration.

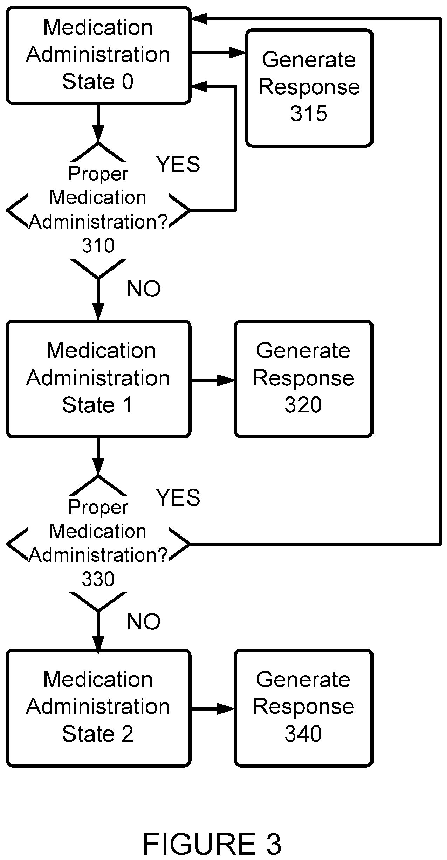

After such a confidence has been determined, as is once again noted in Figure 1, the status of the patient is considered. In general, such status may be based upon various medication administration histories, in addition to other possible inputs to status. Thus, based upon a current status of a patient, proper or improper medication adherence may result in different responses being provided in step 130. Referring therefore to Figure 3, a patient is in a medication administration State 0, preferably indicative of proper prior medication administration or a first use of the system, and including no current medication administration issues. Next, at step 310 a determination of proper medication administration is performed as set forth with respect to the description of Figure 2. If medication administration is determined to be proper, then the patient returns to State 0. A response may be generated at step 315 congratulating the patient for proper medication administration, providing other supportive information, indicating a next time medication is to be taken or the like. Alternatively, even though proper medication administration may have been determined, if a confidence of that administration is determined to be low, or if the patient took a long time to administer the medication, helpful hints or other training suggestions may be provided. Of course, any desired message may be provided.

If at step 310 medication administration is determined to be improper, then the inquiry is answered in the negative, and the patient is placed into medication administration State 1. Based upon programmed thresholds given a desired level of sensitivity (to be described below) a response may be generated at step 320 (corresponding to step 130 in Figure 1). In accordance with various embodiments of the invention, this notification may comprise one or more of an automated reminder to the patient, an automated reminder to a medical professional, a personal contact to the patient from a medical professional or other administrator or care provider, encouraging messages congratulating on a correct administration, thus providing positive reinforcement of proper action by a patient, or no response. The level of such responses may be based upon any number of factors, including but not limited to criticality of adherence to a particular medication protocol, sensitivity of the particular patient or patient population to variations in medication administration timing, dosage or other protocol changes, efficacy of the medication, observable side effects, and the like. Thus, if timing is critical, a first error may generate a personal call from a medical service provider, while if adherence is important over time, but not necessarily for each administration, the patient may only receive an automated message after a first failed administration.

Once in State 1, at the time for a next medication administration, it is determined at step 330 whether such medication administration has been proper. If it is determined that the medication administration has been proper, the patient may be returned to State 0, and one or more responses as noted above may be provided. In an alternative embodiment, it may require more than one consecutive proper administrations to return to State 0, and thus any number of interim states may be provided, with accompanying messages therefrom. From any of these interim states, a failed medication administration may place the patient back into State 1, or other desired state indicative of further administration problems.

If at step 330 it is instead determined that the medication has not been properly administered, and therefore the inquiry at step 330 is answered in the negative, the patient may be placed into medication administration State 2, and a resulting one or more responses may be generated at step 340. It is anticipated that the responses in step 340 in response to the patient being placed in State 2 represent an escalation from the responses provided in step 320. Thus, for example, if the patient received an automated text message in step 320, a personal call from a healthcare provider may be provided in step 340. Any number of responses in step 340 may be provided. Additionally, it is contemplated in accordance with one or more embodiments of the present invention that the system may learn and select a most effective intervention strategy based upon a patient state. Thus, if patients in a particular state most often improve with a particular communication method, but fail to improve with another, the better received communication method may be suggested in the future for communication with one or more patients that are ultimately placed in that same state in the future.

Furthermore, the invention is not so limited to the three states shown in Figure 3. It is anticipated that in accordance with one or more various embodiments of the invention, any number of states may be provided in accordance with one or more medication protocols, thresholds for notifications, and different desired responses to be provided to a patient. Indeed, it is anticipated that as a number of variables being monitored is increased (such as proper administration, time on task variation, or any other measurement associated with proper medication administration, perception of a physical state of the patient with respect to any factor described above and the like), it is likely that the number of states that may be provided is also increased, therefore providing a more customized response to various actions taken by a patient during medication administration.

While the description of Figure 3 has been shown with the determination of medication administration being a binary function, as has been noted above, this need not be the case. Thus, while the apparatus may determine that a user has likely properly administered the medication, the confidence of such a determination may be lower than desirable. It may be possible to define multiple states of the patient, all in response to a determination of a successful administration, but having different levels of confidence in such a determination. Thus, it may be possible to request retraining or otherwise contact the patients, even if there is a determination of proper administration, in order to improve their process for administering the medication.

In particular, many factors may come into play regarding the confidence with which a determination of medication administration may be made. Thus, the detection of certain actions or circumstances of administration by the inventive system may be considered in determining a confidence of administration. Various of these factors may be tracked, and comprise a time sequence of behavioral markers that may also result in desired intervention or other action by the system, in addition to indicating lack of confidence in system determinations. These behavioral markers may comprise any attribute of a patient that may provide insight into a medical condition, or other symptom or the like that may influence a current state of a patient. Furthermore, machine learning of trends may be provided to understanding one or more variables that may aid in best classifying likely candidates, and to allow the system not only to automatically move a patient from state to state, but to define additional states (either as a result of patient improvement or degradation) that may be important to present to a manager or other administrator or care provider to allow for best medication administration monitoring. Various decision fusion learning systems may be employed in order to aid in making determinations regarding the various characteristics that may be reviewed an used to make such state determination decisions.

In one example, consider a patient trying to purposefully fool the system into thinking they have properly administered the medication when in fact they purposefully do not take the medication. During administration, the inventive system preferably watches for a number of predetermined movements, motions or actions performed by a user that may indicate a potential for purposeful tricking of the system. These may include a user's head leaving the display area frequently, the user's hand passing over their mouth during the administration sequence, coughing during the administration sequence, failure of a visible swallowing motion, changes in tone of voice, or any number of additional potential movements or actions. While none of these individually may suggest failure of administration, a number of these, or similar actions over a time sequence may indicate a problem. Therefore, in accordance with various embodiments of the present invention, the occurrence of multiple of such designated actions, or consistent determination one or more of such actions may be tracked, and may be used to place the user in a predefined state requiring further follow up. It is anticipated that such a state may be different from the states noted above where a patient is known to have skipped one or more medication administrations. Thus, any such actions may place the patient in a state or states related to potential malicious behavior, and perhaps requiring further follow up. Thus, whether trying to trick the system or not, embodiments of the present invention may be used to generate a scale of states related to confidence that the person has taken their medication. Once scaled, patients moving into such states over time can be compared to confirm a confidence level of medication administration, and responsive action may be taken by the system, even if it is determined that the person probably did take their medication. These confidence levels may be applied to individual protocols of correct patient behavior.

These confidence levels may further be extended to training to ensure that all steps are confidently completed and allow for comparisons and intervention for improvement. Thus, a patient may be walked through various training sequences in an interactive manner, allowing for direct and near immediate feedback from the system regarding proper medication administration.

In addition to determining whether someone is trying to trick the system, monitoring of various other patient attributes may be employed to give additional insight into patient health, and potentially need for protocol specific, or more general intervention. For example, monitoring of patient attributes, such as change in skin tone or color over time or during different times of the days, visual detection of increased perspiration, visual detection of excessive blinking, head tilting, fidgeting, erratic movement, visual detection of changes in emotion, such as happiness, lethargy, etc., color of whites of eyes, eye movement, pupil dilation, nostril flaring, breathing rates, ticks, twitches, repetition of motion or the like may be employed to indicate a patient positive or adverse reaction to medication administration, lack of medication administration, or simply an overall deterioration or other change in a patient's health over time. Audio clues, such as one or more sounds that may be emitted upon swallowing a pill, for example, may also be employed, in addition to, for example, a swallowing motion of a throat. Thus, audio clues may be used alone, or as a supplement to one or more visual clues. In each of these instances, a baseline attribute measurement of the patient may be determined and used for comparison purposes. Additionally, a time sequence or trend may be determined. In addition to determine changes in such a trend over time, stability over time may be employed as a variable to indicate that any changes from that stable trend should be considered seriously, and may contribute to the movement of a patient from one patient state to another.

In addition to testing and monitoring for general patient attributes, in accordance with another embodiment of the invention, disease or medication specific side effects may be monitored over time. Therefore in accordance with embodiments of the invention, based upon a particular disease state, medication type being administered, or the like, a particular surveillance pattern may be prioritized, and thus the system may look for one or more particular known potential side effects. For example, for an Alzheimer patient, the system might monitor for shaking of a patient's hand, a glass held by the patient or the like. Comparison of shaking over time may be relevant to indicate a deterioration of the condition of the patient over time may be relevant. Acute situations may be determined based upon short term changes in these monitored attributes. Time on task may also be employed in such a situation to further determine deterioration of a patient in a gradual manner, or acutely. Each of these measurements may be employed to influence the patient state, and may in fact result in movement from one state to another, therefore warranting potentially different responses from the system. Furthermore, various implementation of the invention may be employed as a diagnostic tool to help assist care providers in understanding a patient's condition. Thus, patients may be asked one or more questions during or after medication administration, asking or any type of data, and preferably asking for data about a patient's health. Such data may include general wellness questions, or may comprise one or more patient state specific questions regarding side effects, etc. at least in part based upon the state of the patient. Such questions may comprise single answer questions, or may ask for the patient to rate the answers on a scale, as appropriate. Answers to such questions may be used in any desired manner, and may be used to change the state of the patient to provide a more personally tailored medication monitoring and feedback system, to change various dosage and or medication changes, for example.

In addition, while facial and other recognition activities are preferably performed based upon training from a large database, customization of the recognition criteria may be performed based upon one or more captured facial images of a particular user. Thus, rather than determining a particular user emotion, facial expression, or other feature based simply upon a common database, results that may be obtained from such a common database may be enhanced through the use of a weighted average between any of such results, and results primarily, or more weighted, based upon prior use of the system by the particular patient. In such a manner, as a user employs the system, the system will become more familiar with any peculiarities of the particular patient, thus being able to further personalize the system, and determine any changes in patient characteristics (of any type as noted above) to allow for further input into the system to assist with various adherence, training and other medication administration issues, as noted above.

Additionally, audio measurements may be made and monitored as a tracked attribute. Thus, coughing, heavier breathing, stuttering, time for response to audible prompts and the like may be monitored over time, again to determine gradual or acute changes in patient behavior and over time potential changes in the medical state of the patient. A patient may be asked to recite a word sequence, such as their name, so that differences from an expected cadence or the like may be determined. Indeed, it is contemplated in accordance with one or more embodiments of the present invention that if such audio changes in a patient are particularly indicative of changes in particular disease states or medication administration side effects, in addition to simply monitoring such audio responses, a particular audio sequence test may be performed to further test the patient. Such visual and/or audible measurements may further be employed in patients with dementia, distonia, to measure progression of Parkinson's disease, or the like. To further test patients regarding such attributes, the inventive system may purposely change one or more features of a patient medication administration sequence, such as changing a position of placing a pill to judge reaction time, etc. Responses to any of these situations may once again affect the patient's state, thus resulting in different responses and treatment by the system. Thus, the system monitors overall patient adherence, while various of these other attributes, features and the like may be used to adjust responses, and provided potentially different and helpful intervention where appropriate.

More broadly, monitoring of various visual and audible characteristics of the patient and their action may provide insight into progression of disease states, notification of acute or gradual responses to medications, and provide additional input for placing a patient in a particular patient state (see Figure 3), thus resulting in appropriate action being taken by the system regarding intervention, automatically, through a healthcare provider, or other intervention as appropriate. Combinations of such monitored attributes may be employed to generate a multidimensional state picture of a patient, allowing for system response to that particular state. As noted above, any number of such states may be employed, and thus resulting in any number of potential response settings. Furthermore, in accordance with various embodiments of the present invention, it is contemplated to be able to define common and/or typical patient states, and thus variation therefrom in a population. Any such deviation may again result in action taken on behalf of the patient. Additionally, various common states for a particular patient may be determined based upon time of day, or the like, so that comparisons or deviations may be determined from the appropriate particular base state. Much review of such time sequences may be performed in an offline, or non-real time setting, allowing for advanced processing, and then reporting back to the healthcare provider. Thus, such advanced processing need not be performed on a patient's device, but rather can be performed on historical data and be analyzed more completely allowing for advanced processing offline and avoiding placement of a heavy load on small CPUs on the front-end related to various use of video analysis. Visual information that is to be transmitted to a remote location for consideration may be blurred in part or in whole, or using one or more de-identificaiton techniques, such as facial averaging or the like. Additionally, one or more background segmentation and removal techniques may be employed so that the image of the patient may be isolated and identified. Furthermore, one or more identification or face recognition techniques may be employed in order to ensure consistent identity over time and correct identity of the patient, including analysis and identification of one or more care providers or other individuals that may be present in screen. Additionally, pill recognition, facial recognition, other data relevant to direct automated visual observation, and other identification of medication systems, pill orientation, segmentation of pill from surroundings, ratio comparisons of pill size, including one or more edge detection techniques, to further confirm pill identification, facial recognition may be further analyzed in such a manner.

Additionally, it is contemplated that the various thresholds applicable to determine appropriate responses to various patient states may be predetermined, changed by an administrator or the like in order to match a required response by one or more people to actual availability, or may be determined based upon computer learning or the like. Thus, based upon population responses, errors, and states, changes may be made to the notification thresholds. For example, if a particular population continually performs a particular sequence of steps incorrectly, the system may recognize that an automated response, or other response, may cure this issue. Thus, if the system were previously set to send such an automated response after three such errors, it may automatically or manually be retrained to send such an automated response after, for example, a single such error, in order to reduce the overall number of errors. Furthermore, changes may be made to one or more notification thresholds. Finally, through computer learning or other system recommendations, one or more best medical practices for specific populations or patients included in one or more defined patient states, or patient risk factor, may be provided. In addition, consistent failures of performance, or other consistent information may warrant a change of medication dosage or other changes to medication administration protocol for patients in one or more patient states, or may result in reclassification of a state into multiple or a different state based upon divergence of patient responses in a previously classified homogeneous group.

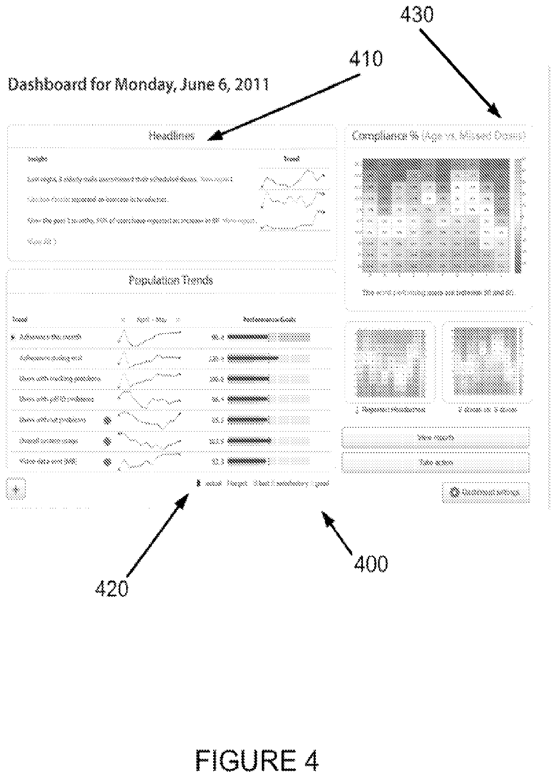

It is preferred that the various information described above be presented to a healthcare provider, clinical trial manager, or other manager of healthcare information in the form of a dashboard providing critical information to allow for review and action related to medication adherence. Therefore, as is shown in the exemplary dashboard display 400 of Figure 4, one or more headlines may be displayed. These headlines are preferably related to adherence characteristics of particular groups of patients, or to individual patients that may need immediate assistance of contact. Of course, a separate display providing a list of patients who may be appropriate for follow up may be provided. Furthermore, a list may be provided indicating all of the individuals who may have been automatically contacted, and a further list of those to be contacted. In accordance with various embodiments of the invention, any changes of state noted above, or potentially available, may become the subject of a headline presented to a manager.

As is further shown in Figure 4, a set of population (or individual) trends 420 may be displayed. Thus, any of the attributes desired to be tracked over time as noted above, may become the subject of a trend report. In this particular display, such trends comprise overall adherence, adherence during a particular trial, users without problems, contraindications, benefits of the medication, etc. Trends for individuals over time may also be displayed. Additionally, as is shown in Figure 4, any number of graphical elements 430 may be displayed, such as histograms, heat maps, or other displays of various adherence information that may be useful to the manager. It is contemplated that such a dashboard may provide any number and variation of information to the manager or other user. In accordance with embodiments of the present invention, patients in different patient states, including placement by any of the means described above, may be displayed in accordance with that state, or in accordance with movement between states, either individually or as part of a group of similarly situated patients. Indeed, it is contemplated that the user may indicate desired information to be displayed, or that the system may track various trend and other information and determine automatically which issues are most critical requiring review by the manager, and display them. Patient touch points and various communications will be logged, stored, integrated into one or more computer learning decisions performed by the system, and made available to the manager through the dashboard. Therefore, the manager may be apprised of issues requiring the most urgent attention. Various additional functionality may be provided on a front or additional page of such a dashboard, displaying further adherence information related to any individual or group.

In addition to providing such a dashboard providing information about a number of patients to a particular healthcare provider, it is anticipates that variations of such a dashboard may be provided in accordance with one or more embodiments of the present invention. Thus, each such administrator may have a unique log in sequence, and thus may be show different information based upon login status, patient accountability, access to confidential information, or preset personalization by the particular user. Thus, in accordance with embodiments of the invention, each use is provided with information relevant to their patient population, and in a format most requested by them. In addition, an individual patient may be provided with a dashboard including information related to their medication administration, including contact information for healthcare providers, scoring for recent adherence administrations, tracking of various attributes or other patient information over time, and any other information relevant to a single patient as described above. Further, direct access to a healthcare provider or the like may be provided, thus allowing for a single stop location for the user to access all medication adherence needs, and allowing different complexities and relevance of various personal adherence information. Of course, use of the system and access to help need not be provided through such a dashboard, and may be provided directly from the medication adherence processing system.

While the present technology has been described related to monitoring medication adherence, it is contemplated in accordance with various embodiments of the invention that the monitoring scheme including video and audio data, and including various computer learning systems for classifying such information, may be applied in various additional areas, such as monitoring manufacturing processes, energy generation and management, and indeed any situation in which automatically determining actions of a person, and providing near real time intervention may be beneficial.

It will thus be seen that the objects set forth above, among those made apparent from the preceding description, are efficiently attained and, because certain changes may be made in carrying out the above method and in the construction(s) set forth without departing from the spirit and scope of the invention, it is intended that all matter contained in the above description and shown in the accompanying drawings shall be interpreted as illustrative and not in a limiting sense.

It is also to be understood that this description is intended to cover all of the generic and specific features of the invention herein described and all statements of the scope of the invention which, as a matter of language, might be said to fall there between.

Embodiments of the present invention may include:-
1. A method for monitoring medication adherence, comprising the steps of: determining a present adherence state of a patient;
   receiving video analysis information reporting on a medication administration session;
   determining a next adherence state of a patient based upon the present adherence state of
   the patient and the video analysis information
2. The method of 1, further comprising the step of initiating one or more actions based upon determination of the next adherence state.
3. The method of 1, further comprising the step of initiating one or more actions based upon a trend from the present adherence state and the next adherence state.
4. The method of 1, wherein the video analysis information includes an automated determination from a video record whether a user has taken a medication.
5. The method of 1, wherein the video analysis information comprises a computer vision analysis of a medication administration session.
6. The method of 1, wherein the video analysis information comprises a trend of a predetermined variable over time.
7. The method of 1, wherein the video analysis information comprises a determination that a particular variable differs by greater than a predetermined threshold amount from one or more prior variable values.
8. The method of 1, wherein a confidence level associated with the video analysis information is employed to determine the next patient adherence state.
9. The method of 8, wherein the confidence level comprises a confidence trend level over time.
10. The method of 1, further comprising the step of displaying the patient adherence state information on a dashboard display.
11. The method of 1, wherein the dashboard display shown a plurality of trends over time determined from the video analysis information.
12. The method of 11, wherein the plurality of trends are used to determine the next patient adherence state.
13. The method of 1, wherein further processing of the video analysis information at a time remote from the time of medication administration is employed at least in part to determine a next adherence state of a patient.
14. The method of 1, wherein one or more common patient states between a plurality of patients are employed to group those patients.
15. The method of 14, wherein the grouped patients may be addressed under a common scheme.
16. The method of 15, wherein the common scheme may be employed to address particular high risk patients.
17. The method of 1, wherein one or more next adherence states are determined through a computer learning analysis of the received video analysis information.
18. The method of 1, further comprising the use of audio information m conjunction with the video information.
19. A system for implementing the method of 1-16.
   Further Embodiments of the present invention may include:-
   1 A method wherein the confidence level comprises a confidence trend level over time.
   2 The method of 1, wherein further processing of the video analysis information at a time remote from the time of medication administration is employed at least in part to determine a next adherence state of a patient.
   3 The method of 1, wherein grouped patients may be addressed under a common scheme.
   4 The method of 3, wherein the common scheme may be employed to address particular high risk patients.
Further Embodiments of the present invention may include:-
1. A method for monitoring medication adherence, comprising the steps of:
   determining a present adherence state of a patient;
   receiving video analysis information reporting on a medication administration session;
   determining a next adherence state of a patient based upon the present adherence state of the patient and the video analysis information
2. The method of 1, further comprising the step of initiating one or more actions based upon determination of the next adherence state.
3. The method of 1, further comprising the step of initiating one or more actions based upon a trend from the present adherence state and the next adherence state.
4. The method of 1, wherein the video analysis information includes an automated determination from a video record whether a user has taken a medication.
5. The method of 1, wherein the video analysis information comprises a computer vision analysis of a medication administration session.
6. The method of 1, wherein the video analysis information comprises a trend of a predetermined variable over time.
7. The method of 1, wherein the video analysis information comprises a determination that a particular variable differs by greater than a predetermined threshold amount from one or more prior variable values.
8. The method of 1, wherein a confidence level associated with the video analysis information is employed to determine the next patient adherence state.
9. The method of 1, further comprising the step of displaying the patient adherence state information on a dashboard display.
10. The method of 1, wherein the dashboard display shown a plurality of trends over time determined from the video analysis information.
11. The method of 10, wherein the plurality of trends are used to determine the next patient adherence state.
12. The method of 1, wherein one or more common patient states between a plurality of patients are employed to group those patients.
13. The method of 1, wherein one or more next adherence states are determined through a computer learning analysis of the received video analysis information.
14. The method of 1, further comprising the use of audio information in conjunction with the video information.
15. A system for implementing the method of 1-14.

## Claims

1. A method for monitoring medication adherence, comprising the steps of:
determining a present adherence state of a patient in accordance with a historical medication adherence profile of the patient, and including at least one or more of a historical track record for proper medication adherence for the patient, whether the patient is in a higher risk group that is likely to have adherence problems, and whether a medication to be administered is in a group requiring critical administration timing;
confirming in near-real time by computer vision technology proper medication administration by the patient by confirming proper patient action, proper placement of a medication for identification of identifying a medication to be taken, such identification confirmed employing one or more of color, bar coding, marking or other identifiable characteristics, and determining a confidence level of proper ingestion of the medication; and
determining a next adherence state of a patient based upon the present adherence state of the patient and the determined confidence level of proper ingestion of the medication.

2. The method of claim 1, further comprising the step of initiating one or more actions based upon determination of the next adherence state.

3. The method of claim 1, further comprising the step of initiating one or more actions based upon a trend from the present adherence state and the next adherence state.

4. The method of claim 1, wherein the confidence level comprises a confidence trend level over time.

5. The method of claim 1, further comprising the step of displaying the patient adherence state information on a dashboard display.

6. The method of claim 5, wherein the dashboard display shown a plurality of trends over time determined from the video analysis information.

7. The method of claim 6, wherein the plurality of trends are used to determine the next patient adherence state.

8. The method of claim 1, wherein further processing by computer vision technology confirming proper medication administration by the patient is performed at a time remote from the time of medication administration is employed at least in part to determine a next adherence state of a patient.

9. The method of claim 1, wherein one or more common patient states between a plurality of patients are employed to group those patients.

10. The method of claim 9, wherein the grouped patients may be addressed under a common scheme.

11. The method of claim 10, wherein the common scheme may be employed to address particular high risk patients.

12. The method of claim 1, wherein one or more next adherence states are determined through a computer learning analysis of the processed information.

13. The method of claim 1, further comprising the use of audio information in conjunction with the video information.

14. A system for monitoring medication adherence, comprising the steps of:
a display;
an image capture device; and
a computer processor configured to:
automatically determine a present adherence state of a patient in accordance with a historical medication adherence profile of the patient, and including at least one or more of a historical track record for proper medication adherence for the patient, whether the patient is in a higher risk group that is likely to have adherence problems, and whether a medication to be administered is in a group requiring critical administration timing;
in response to one or more captured images by the image capture device, confirm in near-real time by computer vision technology proper medication administration by the patient by confirming proper patient action, proper placement of a medication for identification of identifying a medication to be taken, such identification confirmed employing one or more of color, bar coding, marking or other identifiable characteristics, and determining a confidence level of proper ingestion of the medication; and
determine a next adherence state of a patient based upon the present adherence state of the patient and the determined confidence level of proper ingestion of the medication.
